# EUROPÄISCHE PATENTSCHRIFT

(11) **EP 1 128 856 B1**
(45) Veröffentlichungstag und Bekanntmachung des Hinweises auf die Patenterteilung: **29.11.2006**
(21) Anmeldenummer: 99969345.0
(22) Anmeldetag: 20.09.1999
(51) Int. Cl.: A61M 5/172

(54) **INFUSIONSPUMPE MIT EINEM RECHNER ZUM BERECHNEN DER JEWEILS HÖCHSTZULÄSSIGEN ABGABEMENGE**
INFUSION PUMP COMPRISING A COMPUTER FOR CALCULATING THE RESPECTIVE MAXIMUM PERMISSIBLE DOSAGE
POMPE A PERFUSION MUNIE D'UN ORDINATEUR POUR CALCULER CHACUNE DES DOSES ADMISSIBLES A DELIVRER

(30) Priorität: 18.09.1998 DE 19842722
(43) Veröffentlichungstag der Anmeldung: 05.09.2001
(73) Patentinhaber: Codman Neuro Sciences Sàrl, 2400 Le Locle (CH)
(72) Erfinder: TÖNNIES, Jan, G., D-24105 Kiel (DE)
(74) Vertreter: Bardehle, Heinz
(86) Internationale Anmeldenummer: PCT/DE1999/003000
(87) Internationale Veröffentlichungsnummer: WO 2000/016823

(56) Entgegenhaltungen:
- WO-A-84/03218
- GB-A- 2 153 081
- US-A- 4 475 901
- US-A- 5 010 473
- US-A- 5 800 387
- MARTIN R W ET AL: "AN OPEN-LOOP COMPUTER-BASED DRUG INFUSION SYSTEM" IEEE TRANSACTIONS ON BIOMEDICAL ENGINEERING,US,IEEE INC. NEW YORK, Bd. BME-34, Nr. 8, 1. August 1987 (1987-08-01), Seiten 642-648, XP000028128 ISSN: 0018-9294

## Beschreibung

Die Erfindung betrifft eine Infusionspumpe zum Abgeben einer über ein elektronisches Steuergerät bestimmbaren Menge eines Medikaments in den Körper eines Patienten, wobei die Pumpe mit einem Rechner zum Berechnen der jeweils höchstzulässigen Abgabemenge in Abhängigkeit von der zuvor abgegebenen Menge und einer Sperreinrichtung zum Verhindern der weiteren Abgabe des Medikaments bei Überschreiten eines vorgegebenen zulässigen Maximalwerts versehen ist versehen ist.

Derartige Infusionspumpen dienen dazu, einen Patienten über längere Zeit mit einem Medikament versorgen zu können, wobei die von der Infusionspumpe dem jeweiligen Bedarf des Patienten entsprechend kontinuierlich abgegebene Menge des Medikaments eingestellt werden kann.

Der Oberbegriff des Anspruchs 1 ist aus der DE 33 90 462 C2 bekannt, welche eine implantierbare Infusionspumpe zeigt, die mit einem Rechner versehen ist, der über ein "gleitendes Zeitfenster", beispielsweise über drei Stunden, die jeweils abgegebene Medikamentenmenge bestimmt und dann, wenn die über diesen Zeitraum abgegebene Menge einen Höchstwert übersteigt, die weitere Abgabe sperrt.

Dieses Vorgehen ist aber ist unzulänglich, die Länge des gleitenden Zeitfensters willkürlich. In manchen Fällen - etwa bei einem Schmerzanfall - ist es erforderlich, die Menge des von der Pumpe abzugebenden Wirkstoffs kurzzeitig erheblich zu erhöhen, um den Wirkstoffpegel schnell anzuheben. Eine Menge, die über drei Stunden verteilt tolerabel ist, kann bei einer Gabe über drei Minuten aber toxisch sein. Andererseits aber kann eine Infusionsrate, die über drei Stunden verteilt zulässige ist, bei einer über drei Stunden hinausgehenden Gabe toxisch oder gar letal sein. Dieses Problem ist mit dem "gleitenden Zeitfenster" nicht zu lösen.

Der Erfindung liegt die Aufgabe zugrunde, eine implantierbare Infusionspumpe zu schaffen, die es erlaubt, die jeweils zulässige Abgabemenge in zuverlässiger Weise zu bestimmen.

Erfindungsgemäß wird diese Aufgabe dadurch die Merkmale des Anspruchs 1 gelöst.

Der Maximalwert, bei dem das Sperren erfolgt, ist damit nicht wie bei dem Stand der Technik eine über ein bestimmmtes Zeitfenster gemittelter Wert, sondern ergibt sich als das um den sich aus der Halbwertzeit des Medikament ergebenden Betrag geminderten Integrals über die insgesamt abgegebene Menge.

Dabei ist der Rechner vorzugsweise mit einer Einrichtung versehen, die entweder mit einem dem erwarteten Abbau des Medikaments im Körper entsprechenden vorgegebenen Zeitabstand die Subtraktion eines bestimmten prozentualen Anteils des jeweils in den Speicher eingeschriebenen Betrages, oder aber mit fest vorgebenen Zeitabständen die Subtraktion eines dem erwarteten Abbau des Medikaments entsprechenden prozentualen Anteils des jeweils in den Speicher eingeschriebenen Betrages bewirkt.

Bei der vorgeschlagenen Ausbildung der Infusionspumpe ist sichergestellt, daß die Medikamentengabe, die über das Steuergerät von dem Arzt oder ggf. auch von dem Patienten selbst bewirkt wird, einen maximal zulässigen Höchstwert nicht übersteigt.

Um das Gerät für einen bestimmten Patienten einzustellen, bedarf es lediglich der Eingabe der dem zu verabreichenden Medikament eigenen Halbwertzeit und des individuell zulässigen Maximalwerts (der toxischen Schwelle).

Es versteht sich, daß das Gerät auch so prammierbar ausgebildet sein kann, daß die Einhaltung eines unteren Minimalwerts (der Wirkschwelle) gewährleistet ist.

Bei der Pumpe kann es sich auch um eine implantierbare Infusionspumpe handeln. Dabei kann der Rechner (oder aber ein zusätzlicher, parallel arbeitender Rechner) auch in einem externen Steuergerät angeordnet sein. Es kann dabei vorgesehen sein, daß eine Bolusgabe (nämlich eine Infusion des Medikaments, die bei einer dauerhaften Gabe zu einer Überschreitung des toxischen Schwellenwerts führen wurde) nur bei (elektromagnetischer) Kopplung mit dem Steuergerät möglich ist.

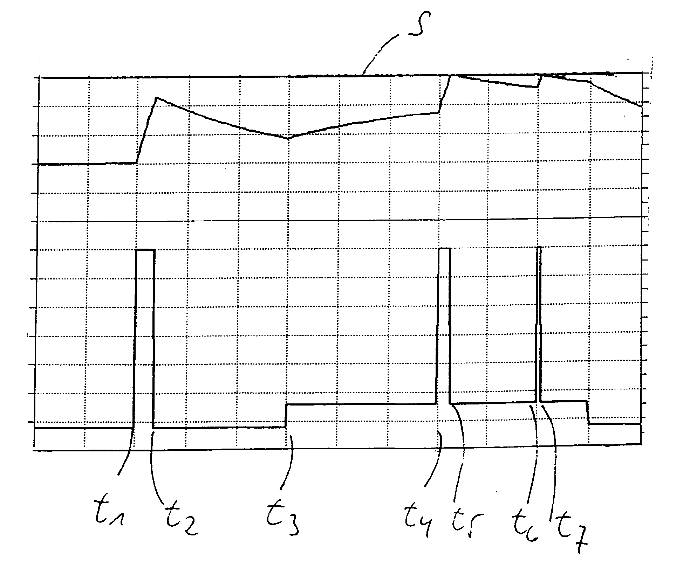

Die Erfindung wird im folgenden anhand einer Zeichnung erläutert. Dabei zeigt die einzige Figur in dem unteren Graph ein Infusionsprofil und in dem oberen Graph einen sich aus diesem Infusionsprofil ergebenden Verlauf des jeweils in den Speicher eingeschriebenen Betrages, der der erwarteten Menge (und damit der Konzentration) des sich jeweils in dem Körper des Patienten befindenden Wirkstoffs entspricht, sowie den vorgebenen zulässigen Maximalwert (Schwellenwert S).

Bei dem dargestellten Infusionsprofil erfolgt zunächst eine Dauergabe mit einer relativ geringen Infusionsrate. Vom Zeitpunkt t1 bis t2 erfolgt (durch den Patienten oder einen Arzt verursacht) eine erste Bolusgabe, also eine kurzzeitige Gabe mit einer hohen Infusionsrate, wie sie beispielsweise bei einem akuten Anfall des Patienten erforderlich ist. Zum Zeitpunkt t3 erfolgt eine Umschaltung auf eine höhere Infusionsrate. Zum Zeitpunkt t4 wird wieder über das Steuergerät die Gabe eines Bolus bewirkt, der aber bei Erreichen des vorgegebenen Schwellenwerts S von dem Rechner vorzeitig zum Zeitpunkt t5 abgebrochen wird. Zum Zeitpunkt t6 wird Versuch des Verwenders, erneut eine Bolusgabe zu setzen, alsbald, nämlich schon zum Zeitpunkt t7, abgebrochen, weil der Schwellenwert S erreicht worden ist.

Der sich aus diesem Infusionsprofil ergebende Verlauf der Wirkstoffkonzentration im Körper des Patienten (die im wesentlichen proportional zu der im Körper jeweils vorhandenen Wirkstoffmenge ist) ist in dem unteren Graph dargestellt.

Der Verlauf der Wirkstoffkonzentration stellt sich als zeitliches Integral über die infundierte Menge, vermindert um die sich aus dem sich aus der Halbwertzeit des Wirkstoff ergebenden Abbau dar, also als eine Funktion mit einem - von der Gabe des Medikaments bestimmten - linearen Glied und einem - von der Abbaurate des Medikaments bestimmten - negativen Exponential-Glied.

Dies führt in der Figur bis zum Zeitpunkt t1 zu einem gleichbleibenden Verlauf, weil hier die jeweils zugeführte Menge der von dem Körper abgebauten Menge genau entspricht. Die Gabe des Bolus zum Zeitpunkt t1 führt zu einem steilen Anstieg der Wirkstoffkonzentration. Nach Beendigung der Gabe des Bolus zum Zeitpunkt t2 fällt die Konzentration kontinuierlich ab, weil die zugeführte Wirkstoffmenge geringer als die abgebaute Menge ist. Nach der Verdoppelung der Infusionsrate zum Zeitpunkt t3 steigt die Konzentration stetig, aber mit sich abflachender Steigung an.

Die Bewirkung einer erneuten Bolusgabe durch den Verwender (oder den Arzt) zum Zeitpunkt t4 führt zu einem Anstieg der Konzentration bis auf den Schwellenwert zum Zeitpunkt t5, was zum Zeitpunkt t6 einer automatischen Beendigung der Bolusgabe durch den Rechner führt. Der zum Zeitpunkt t7 erfolgende Versuch einer erneuten Bolusgabe wird von dem Rechner wegen alsbaldigem Erreichen des Schwellenwerts sogleich verhindert.

Der Verlauf der Wirkstoffkonzentration wird in dem Rechner der implantierbaren Infusionspumpe (der auch in dessen Steuergerät angeordnet sein kann) simuliert:

In vorgegebenen Zeitabständen - beispielsweise alle 10 sec - wird der in dem Speicher der Infusionspumpe eingeschriebene Betrag um einen der von der Infusionspumpe in diesem Zeitraum abgegebenen Menge entsprechenden Betrag erhöht. Weiter wird ein sich aus der Halbwertzeit des abgegebenen Medikaments rechnerisch ergebender prozentualer Anteil des in den Speicher eingeschriebener Betrag subtrahiert, der sich ergebende Betrag wird als aktueller Wert abgespeichert (alternativ kann auch in sich aus der Halbwertzeit ergebenden Zeitanständen (also bei einer kürzeren Halbwertzeit häufiger, bei einer größeren Halbwertzeit seltener) die in diesem Zeitraum abgegebene Menge aufaddiert und ein fester Betrag subtrahiert werden).

Der in den Speicher jeweils eingeschriebene Wert entspricht damit immer (wegen der nicht genau bestimmbaren Halbwertzeit natürlich nur näherungsweise) der jeweiligen tatsächlichen Menge (bzw. Konzentration) des Wirkstoffs im Körper des Patienten unter Berücksichtigung dessen Abbaus.

## Patentansprüche

1. Infusionspumpe zum Abgeben einer über ein elektronisches Steuergerät bestimmbaren Menge eines Medikaments in den Körper eines Patienten, wobei die Pumpe mit einem Rechner zum Berechnen der jeweils höchstzulässigen Abgabemenge in Abhängigkeit von der zuvor abgegebenen Menge und mit einer Sperreinrichtung zum Verhindern der weiteren Abgabe des Medikaments bei Überschreiten eines vorgegebenen zulässigen Maximalwerts versehen ist, **dadurch gekennzeichnet, dass** der Rechner die sich aus der abgegebenen Menge des Medikaments und dessen Abbau in dem Körper ergebende jeweilige Menge oder Konzentration des Wirkstoffs in dem Körper des Patienten bestimmt und diesen mit dem vorgegebenen Maximalwert vergleicht, und **dadurch** dass der Rechner
- einen Speicher, in den ein Betrag eingespeichert ist, der sich durch Aufaddieren der jeweils abgegebenen Menge und Subtrahieren des sich aus dem erwarteten Abbau des Medikaments in dem Körper ergebenden prozentualen Anteiles des jeweils in dem Speicher eingeschriebenen Betrages ergibt, und
- einen Komparator, der den in dem jeweils in den Speicher eingeschriebenen Betrag ständig mit dem vorgegebenen zulässigen Maximalwert vergleicht,
aufweist, wobei vorstehendes Aufaddieren und Subtrahieren als auch das Vergleichen mit dem Maximalwert in vorgegebenen Zeitabständen erfolgt.

2. Infusionspumpe nach Anspruch 1, **dadurch gekennzeichnet, dass** der Zeitabstand dem erwarteten Abbau des Medikaments im Körper entspricht.

3. Infusionspumpe nach Anspruch 1, **dadurch gekennzeichnet, dass** der Zeitabstand fest vorgegeben ist.

4. Infusionspumpe nach einem der vorangehenden Ansprüche, **gekennzeichnet durch** die Ausbildung als implantierbare Infusionspumpe mit externem Steuergerät.

5. Infusionspumpe nach Anspruch 4, **dadurch gekennzeichnet, dass** ein erster Rechner in der Infusionspumpe und ein zweiter in dem Steuergerät angeordnet ist.

## Claims

1. Infusion pump for the delivery of an amount of a medicament to the body of a patient determinable by means of an electronic control device, the pump being provided with a computer for calculating the in each case maximum permitted administration quantity as a function of the previously delivered quantity and with a blocking device for preventing further administration of the medicament on exceeding a predetermined, permitted maximum value, **characterized in that** the computer determines the amount or concentration of the active substance in the body of the patient resulting from the delivered medicament quantity and its breaking down in the body and compares it with the predetermined maximum value, and that the computer is provided with
- a memory in which is stored a quantity resulting from the adding up of the in each case delivered amount and subtracting the percentage of the quantity entered in each case in the memory resulting form the expected breaking down of the medicament in the body, and
- a comparator which compares the quantity entered in each case in the memory constantly with the predetermined, permitted maximum value,
and said adding up and subtracting as well as comparing with the maximum value occurs in predetermined time intervals.

2. Infusion pump according to claim 1, **characterized in that** the time interval corresponds to the expected breaking down of the medicament in the body.

3. Infusion pump according to claim 1, **characterized in that** the time interval is fixedly predetermined.

4. Infusion pump according to one of the preceding claims, **characterized by** its construction as an implantable infusion pump with an external control device.

5. Infusion pump according to claim 4, **characterized by** a first computer in the infusion pump and a second computer in the control device.

## Revendications

1. Pompe à perfusion pour délivrer dans le corps d'un patient une quantité de médicament déterminée par l'intermédiaire d'un appareil de commande électronique, la pompe comprenant un ordinateur pour calculer à chaque fois la quantité maximale admissible à délivrer en fonction de la quantité préalablement délivrée et comprenant un dispositif d'arrêt pour empêcher la poursuite de la délivrance du médicament en cas de dépassement d'une valeur maximale admissible prédéfinie, **caractérisée en ce que** l'ordinateur détermine la quantité ou la concentration de principe actif dans le corps du patient sur la base de la quantité de médicament délivrée et de sa dégradation dans le corps du patient et compare cette valeur à la valeur maximale prédéfinie, et **en ce que** l'ordinateur comprend
- une mémoire où est mémorisée une grandeur obtenue par addition de la quantité délivrée à chaque fois et par soustraction du pourcentage, par rapport à la grandeur enregistrée à chaque fois dans la mémoire, de la dégradation escomptée du médicament dans le corps, et
- un comparateur qui compare en permanence la grandeur enregistrée dans la mémoire et la valeur maximale admissible prédéfinie,
lesdites addition et soustraction ainsi que la comparaison avec la valeur maximale s'effectuant à des intervalles de temps prédéfinis.

2. Pompe à perfusion selon la revendication 1, **caractérisée en ce que** l'intervalle de temps correspond à la dégradation escomptée du médicament dans le corps.

3. Pompe à perfusion selon la revendication 1, **caractérisée en ce que** l'intervalle de temps est prédéfini de manière fixe.

4. Pompe à perfusion selon une des revendications précédentes, **caractérisée en ce qu'**elle est conçue sous la forme d'une pompe à perfusion implantable avec appareil de commande extérieur.

5. Pompe à perfusion selon la revendication 4, **caractérisée en ce qu'**un premier ordinateur est disposé dans la pompe à perfusion et un second dans l'appareil de commande.
